# EUROPEAN PATENT APPLICATION

(11) **EP 4 548 844 A1**
(43) Date of publication of application: **07.05.2025**
(21) Application number: 23846976.1
(22) Date of filing: 25.07.2023
(51) Int. Cl.: A61B 5/145, A61B 5/346, A61B 5/00, G16H 50/20

(54) **METHOD FOR MEASURING REAL-TIME VALUES AND MANAGEMENT OF BLOOD SUGAR ON BASIS OF ELECTROCARDIOGRAM, AND COMPUTER PROGRAM RECORDED ON RECORDING MEDIUM IN ORDER TO EXECUTE SAME**

(30) Priority: 29.07.2022 KR 20220094662
(71) Applicant: Medical AI Co., Ltd., Seoul 06180 (KR)
(72) Inventor: KWON, Joonmyoung, Seoul 06180 (KR)
(74) Representative: Patentanwaltskanzlei Matschnig & Forsthuber OG
(86) International application number: PCT/KR2023/010775
(87) International publication number: WO 2024/025318

(57) **Abstract**

The present invention proposes a method of measuring the real-time level and degree of management of blood sugar based on an electrocardiogram. The method includes: obtaining an electrocardiogram signal measured for a user; estimating the user's blood sugar level by analyzing the electrocardiogram signal using a pre-trained first artificial intelligence, and determining whether the user has hyperglycemia or hypoglycemia by analyzing the electrocardiogram signal using an artificial intelligence; and determining whether the user needs guidance on blood sugar or diabetes based on the estimated blood sugar level and the result of the determination of whether the user has hyperglycemia or hypoglycemia. Through this, a user may check the user's immediate blood sugar level and how well the blood sugar is managed simply by measuring the electrocardiogram 10, and as a result, hyperglycemia and diabetes may be detected early or a complication attributable to hyperglycemia or diabetes may be prevented.

## Description

### Technical Field

The present invention relates to smart health care, and more specifically, to a method capable of measuring the real-time level and degree of management of blood sugar based on an electrocardiogram (ECG), and a computer program recorded on a storage medium in order to execute the same.

### Background Art

An electrocardiogram is a graphical recording of visual changes in electrical activity generated by the heart muscles. Briefly, as for the conduction system of the heart, the sinoatrial node (the SA node) periodically generates an electrical signal to induce the contraction of the heart. When the electrical signal generated by the sinoatrial node (the SA node) is transmitted through the atrium, the atrioventricular node (the AV node) slightly delays the electrical signal and then transmits it. The electrical signal transmitted from the AV node spreads throughout the ventricle through the bundle of His and Purkinje fibers.

As for the waveforms of an electrocardiogram that can be identified according to the process of conduction of the heart, the P wave can be identified during the process in which as the atrium becomes polarized by the electrical signal generated by the sinoatrial node (the SA node), the heart muscles of the valve contract, and, as the atrium becomes depolarized again, the heart muscles of the valve relax again. The P-Q wave can be identified during the process in which the AV node delays the electrical signal so that the ventricle does not immediately respond when the atrium contracts. The QRS complex can be identified during the process in which the electrical signal delayed by the AV node is transmitted (Q), and thus, the ventricle becomes polarized (R) immediately and then depolarized (S) immediately. The S-T wave can be identified during the resting phase in which the electrical signal does not stimulate the heart so that the ventricle contracts and the blood in the ventricle moves to various parts of the body. Furthermore, the T wave can be identified during the process in which as the ventricle is weakly polarized and then depolarized again, the heart muscles of the ventricle and the valve relax simultaneously. Furthermore, in some cases, the U wave can be identified due to the repolarization of the intraventricular septum.

The order of appearance, shapes, sizes, and intervals of the waveforms (the P wave, the P-Q wave, the QRS wave, the S-T wave, the T wave, and the U wave) that can be identified through the electrocardiogram may change in various manners depending on the health condition. In other words, by analyzing the order of appearance, shapes, sizes, and intervals of the waveforms included in the electrocardiogram, a health condition can be inferred, and even the various causes that cause such a health condition can be inferred.

Meanwhile, Artificial Intelligence (AI) refers to a technology that artificially implements some or all of the learning ability, reasoning ability, and perception ability of humans using a computer program. In connection with artificial intelligence, machine learning refers to the learning that is performed to optimize parameters with given data by using a model composed of multiple parameters.

Such machine learning is divided into supervised learning, unsupervised learning, and reinforcement learning according to their learning methodology. Furthermore, machine learning is divided into regression analysis, an artificial neural network (ANN), and deep learning (DL) according to their learning algorithm.

On the other hand, blood sugar refers to glucose contained in the blood. Generally, a blood sugar level is maintained at a constant level by the interaction of insulin, glucagon, epinephrine, glucocorticoid, adrenocorticotrophic hormone, and thyroid hormone. Furthermore, diabetes mellitus (DM) is a metabolic disease in which symptoms such as hyperglycemia appear due to the insufficient secretion of insulin or the abnormal functioning of secreted insulin.

When hyperglycemia persists for a long time or diabetes becomes severe, myocardial infarction caused by the acute blockage of a coronary artery or stroke caused by the blockage or rupture of a cerebral artery may occur, and a complication such as amblyopia, caecitas, renal function impairment, or the like may also occur.

According to various research results, it has been revealed that when the level of glycated hemoglobin (HbA1c), which means hemoglobin with glucose bound to it, is lowered by 1%, the risk of a complication attributable to hyperglycemia or diabetes is reduced by about 20% to 30%. Therefore, there is a demand for various means capable of supporting thorough blood sugar management before a complication attributable to hypertension or diabetes occurs.

### Disclosure

### Technical Problem

One object of the present invention is to propose a method capable of measuring the real-time level and degree of management of blood sugar based on an electrocardiogram.

Another object of the present invention is to propose a computer program recorded on a storage medium in order to execute a method capable of measuring the real-time level and degree of management of blood sugar based on an electrocardiogram.

The objects of the present invention are not limited to the objects mentioned above, and other objects not mentioned will be clearly understood by those skilled in the art from the following description.

### Technical Solution

In order to accomplish the above-described object, the present invention proposes a method of measuring the real-time level and degree of management of blood sugar. The method includes: obtaining an electrocardiogram signal measured for a user; estimating the user's blood sugar level by analyzing the electrocardiogram signal using a pre-trained first artificial intelligence, and determining whether the user has hyperglycemia or hypoglycemia by analyzing the electrocardiogram signal using an artificial intelligence; and determining whether the user needs guidance on blood sugar or diabetes based on the estimated blood sugar level and the result of the determination of whether the user has hyperglycemia or hypoglycemia.

The method may further include, after determining whether the user needs guidance, when it is determined that the user needs guidance, transmitting information about guidance, set based on the blood sugar level, the glycated hemoglobin (HbA1c) level, whether the user has hyperglycemia or hypoglycemia, and/or whether the glycated hemoglobin (HbA1c) is normal or abnormal, to user equipment (UE) preset to correspond to the user.

Furthermore, determining whether the user has hyperglycemia or hypoglycemia may include estimating the user's glycated hemoglobin (HbA1c) level by analyzing the electrocardiogram signal using a pre-trained second artificial intelligence and determining whether glycated hemoglobin (HbA1c) is normal or abnormal by analyzing the electrocardiogram signal using the second artificial intelligence.

More specifically, determining whether the user has hyperglycemia or hypoglycemia may include: extracting at least one piece of feature information by decomposing the obtained electrocardiogram signal based on at least one of amplitude, frequency, and time; segmenting the extracted feature information into a plurality of segmentation units according to the time-series order in which the electrocardiogram signal was measured; obtaining pluralities of level values and probability values from the first and second artificial intelligences by inputting the plurality of segmentation units into each of the first and second artificial intelligences; and estimating the blood sugar level and determining whether the user has hyperglycemia or hypoglycemia based on the level values and probability values obtained from the first artificial intelligence, and estimating the glycated hemoglobin (HbA1c) level and determining whether glycated hemoglobin (HbA1c) is normal or abnormal based on the level values and probability values obtained from the second artificial intelligence.

In this case, extracting the feature information may include extracting the feature information by decomposing the electrocardiogram signal using one of wavelet transform, Ensemble Empirical Mode Decomposition (EEMD), Triadic Motif Field (TMF), and Triadic Motif Difference Field (TMDF).

According to one embodiment, segmenting the extracted feature information into the plurality of segmentation units may include segmenting the feature information, obtained from the electrocardiogram signal, into a plurality of segmentation units each having a size corresponding to that of a window while sliding the window having a preset size along a time axis.

Meanwhile, obtaining the pluralities of level values and probability values may include obtaining the pluralities of level values and probability values by inputting feature vectors for the plurality of segmentation units to each of the multiple linear regression and classification models of the first and second artificial intelligences.

Determining whether the glycated hemoglobin (HbA1c) is normal or abnormal may include removing outliers, included in the level values obtained from the first and second artificial intelligences, based on a preset validated range, estimating the blood sugar level by using the result of soft voting based on an average value of level values from which outliers have been removed among the level values obtained from the first artificial intelligence, and estimating the glycated hemoglobin (HbA1c) level by using the result of soft voting based on an average value of level values from which outliers have been removed among the level values obtained from the second artificial intelligence.

Determining whether the glycated hemoglobin (HbA1c) is normal or abnormal may include removing outliers, included in the probability values obtained from the first and second artificial intelligences, based on a preset validated range, determining whether the user has hypoglycemia or hyperglycemia by using the result of hard voting based on a majority of level values from which outliers have been removed among the probability values obtained from the first artificial intelligence, and determining whether the glycated hemoglobin (HbA1c) is normal or abnormal by using the result of hard voting based on a majority of probability values from which outliers are removed among the probability values obtained from the second artificial intelligence.

In order to accomplish the above-described object, the present invention proposes a computer program recorded on a storage medium in order to execute a method of measuring the real-time level and degree of management of blood sugar based on an electrocardiogram. The computer program is coupled to a computing device, including: memory; a transceiver; an input/output device; and a processor for processing instructions loaded into the memory. Furthermore, the compute program may be a computer program recorded on a storage medium in order to execute: obtaining, by the processor, an electrocardiogram signal measured for a user via the transceiver or input/output device; estimating, by the processor, the user's blood sugar level by analyzing the electrocardiogram signal using a pre-trained first artificial intelligence, and determining, by the processor, whether the user has hyperglycemia or hypoglycemia by analyzing the electrocardiogram signal using an artificial intelligence; and determining, by the processor, whether the user needs guidance on blood sugar or diabetes based on the estimated blood sugar level and the result of the determination of whether the user has hyperglycemia or hypoglycemia.

Specific details of other embodiments are included in the detailed description and the drawings.

### Advantageous Effects

According to embodiments of the present invention, a user may check the user's immediate blood sugar level and how well the blood sugar is managed simply by measuring an electrocardiogram. As a result, hyperglycemia and diabetes may be detected early or a complication attributable to hyperglycemia or diabetes may be prevented.

The effects of the present invention are not limited to the effect mentioned above, and other effects not mentioned will be clearly understood by those skilled in the art, to which the present invention pertains, from the description of the claims.

### Description of Drawings

FIG. 1 is an exemplary diagram illustrating a blood sugar management means according to one embodiment of the present invention;
FIG. 2 is an exemplary diagram illustrating a blood sugar management system according to one embodiment of the present invention;
FIG. 3 is a diagram of the logical configuration of a blood sugar management server according to one embodiment of the present invention;
FIG. 4 is an exemplary diagram illustrating a process of training an artificial intelligence (AI) according to one embodiment of the present invention;
FIG. 5 is an exemplary diagram illustrating a method of releasing an electrocardiogram signal according to one embodiment of the present invention;
FIGS. 6 and 7 are exemplary diagrams illustrating processes of performing segmentation into a plurality of segmentation units according to some embodiments of the present invention;
FIG. 8 is an exemplary diagram illustrating processes of performing analysis using artificial intelligence according to some embodiments of the present invention;
FIGS. 9 and 10 are exemplary diagrams illustrating a process of interpreting the analysis results of an artificial intelligence (AI) according to one embodiment of the present invention;
FIG. 11 is a diagram of the hardware configuration of a blood sugar management server according to one embodiment of the present invention; and
FIG. 12 is a flowchart illustrating a method of measuring the real-time level and degree of management of blood sugar according to one embodiment of the present invention.

### Mode for Invention

It should be noted that the technical terms used herein are used only to describe specific embodiments and are not intended to limit the present invention. Furthermore, unless specifically defined otherwise herein, the technical terms used herein should be interpreted as having meanings generally understood by a person having ordinary skill in the art to which the present invention pertains, but should not be interpreted in an excessively comprehensive or excessively narrow sense. Furthermore, when the technical terms used herein are incorrect technical terms that do not accurately represent the spirit of the present invention, they should be replaced with and understood as technical terms that can be correctly understood by a person skilled in the art. Furthermore, the general terms used herein should be interpreted according to the definitions of dictionaries or according to the context, but should not be interpreted in an excessively narrow sense.

In addition, the singular expressions used herein include plural expressions unless the context clearly indicates otherwise. In the present application, the terms "include" or "have" should not be construed as necessarily including all of the various components or various steps described herein, and some of the components or steps may not be included or one or more additional components or steps may be included.

In addition, the terms including ordinal numbers, such as first, second, etc., used herein may be used to describe various components, but the components should not be limited by the terms. The terms are each used only for the purpose of distinguishing one component from another. For example, without departing from the scope of the present invention, the first component may be named the second component, and similarly, the second component may also be named the first component.

When a component is referred to as being "connected" or "coupled" to another component, it may be directly connected or coupled to the other component, but there may be another component therebetween. In contrast, when a component is referred to as being "directly connected" or "directly coupled" to another component, it should be understood that there are no other component therebetween.

Preferred embodiments according to the present invention will be described in detail with reference to the accompanying drawings below. Regardless of drawing numbers, identical or similar components will be given the same reference numerals and redundant descriptions thereof will be omitted. Furthermore, in the description of the present invention, when it is determined that a detailed description of a related known technology may obscure the gist of the present invention, the detailed description will be omitted. Furthermore, it should be noted that the accompanying drawings are only intended to facilitate the easy understanding of the spirit of the present invention and the spirit of the present invention should not be construed as being limited by the accompanying drawings. The spirit of the present invention should be construed as extending to all modifications, equivalents, and substitutes as well as the accompanying drawings.

As described above, when hyperglycemia persists for a long time or diabetes becomes severe, myocardial infarction caused by the acute blockage of a coronary artery or stroke caused by the blockage or rupture of a cerebral artery may occur, and a complication such as amblyopia, caecitas, renal function impairment, or the like may also occur. Accordingly, there is a demand for various means that can support thorough blood sugar management before a complication such as hypertension, diabetes, or the like occurs.

In order to respond to this demand, the present invention proposes a means capable of measuring the real-time level and degree of management of blood sugar based on an electrocardiogram (ECG).

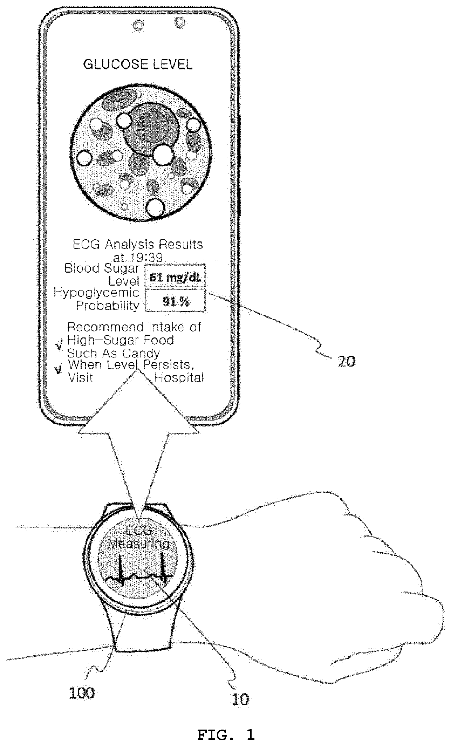

FIG. 1 is an exemplary diagram illustrating a blood sugar management means according to one embodiment of the present invention.

As shown in FIG. 1, the blood sugar management means according to the one embodiment of the present invention measures a user's electrocardiogram 10 using an electrocardiogram reader 100, estimates the user's blood sugar level, glycated hemoglobin (HbA1c) level, and/or the like by analyzing the user's measured electrocardiogram 10 using artificial intelligences (AIs), and provides the user with guidance 20 on blood sugar or diabetes based on the results of the estimation.

Accordingly, according to the one embodiment of the present invention, the user may check the user's immediate blood sugar level and how well the blood sugar is managed simply by measuring the electrocardiogram 10, and as a result, hyperglycemia and diabetes may be detected early or a complication attributable to hyperglycemia or diabetes may be prevented.

Apparatuses and methods for implementing the above-described features will be specifically described below.

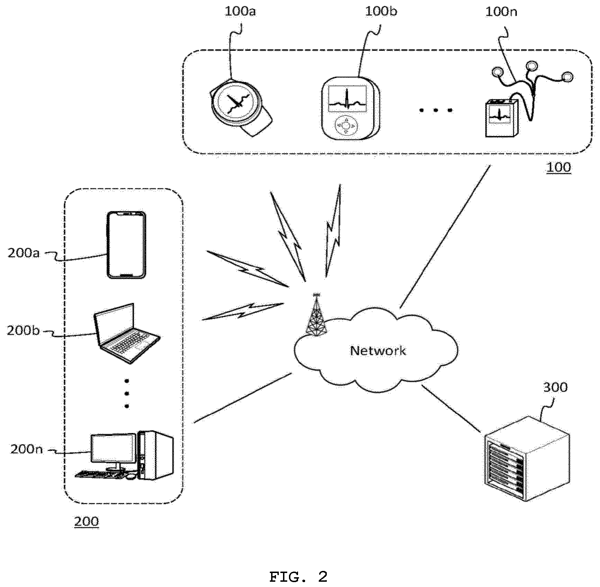

FIG. 2 is an exemplary diagram illustrating a blood sugar management system according to one embodiment of the present invention.

As shown in FIG. 2, the blood sugar management system according to the one embodiment of the present invention may be configured to include one or more electrocardiogram readers 100a, 100b, ···, and 100n (100), one or more pieces of user equipment 200a, 200b, ···, and 200n (200), and a blood sugar management server 300.

The components of the blood sugar management system according to the one embodiment of the present invention are merely functionally distinct components, so that two or more components may be implemented as an integrated component in an actual physical environment or one component may be implemented as separate components in an actual physical environment.

As for the individual components, each of the electrocardiogram readers 100 is a device capable of measuring and recording a user's electrocardiogram 10.

More specifically, each of the electrocardiogram readers 100 may induce changes in electric potential attributable to the electrical activity of the heart muscles via electrodes in contact with the user's body, may amplify the induced changes in electric potential, and may record them as waves.

The electrocardiogram reader 100 according to one embodiment of the present invention may induce an electrocardiogram according to any one of standard limb leads, unipolar limb leads, and precordial leads. The electrocardiogram (10) induction method of the electrocardiogram reader 100 is not limited thereto. The electrocardiogram reader 100 may be equipped with electrodes for a 1 lead, 6 leads, or 12 leads, and the number of leads of the electrocardiogram reader 100 is not limited thereto. Furthermore, the electrocardiogram reader 100 may have any one of the watch type 100a, the portable type 100b, and the holter type 100n, and the type of electrocardiogram reader 100 is not limited thereto.

An electrocardiogram signal (i.e., a recorded waveform signal) measured by the electrocardiogram reader 100 may be directly transmitted to the blood sugar management server 300 through the network function of the electrocardiogram reader 100 itself, or may be input to the blood sugar management server 300 via a third means. For example, the third means may be any one of a removable medium, the user equipment 200, and a data input action performed by a user, but is not limited thereto.

As the next component, the user equipment 200 may output information about the guidance 20 on blood sugar or diabetes determined by the blood sugar management server 300.

More specifically, the user equipment 200 may receive the information about the guidance 20 on blood sugar or diabetes from the blood sugar management server 300. Furthermore, the user equipment 200 may output the received information about the guidance 20 on blood sugar or diabetes.

In this case, the information about the guidance 20 on blood sugar or diabetes may directly include the blood sugar level, the glycated hemoglobin (HbA1c) level, the result of whether the blood sugar is high or low, and/or the result of whether the glycated hemoglobin (HbA1c) is normal or abnormal, or may include indirect precautions, recommendations, and/or warnings based on the blood sugar level, the glycated hemoglobin (HbA1c) level, the result of whether the blood sugar is high or low, and/or the result of whether the glycated hemoglobin (HbA1c) is normal or abnormal. Furthermore, the information about the guidance 20 on blood sugar or diabetes may include at least one of guidance on actions that the user has to take immediately in connection with blood sugar or diabetes and guidance intended to support the user's diabetes management.

The user equipment 200 according to one embodiment of the present invention is not limited to user equipment (UE) defined by the 3rd Generation Partnership Project (3GPP) or a mobile station (MS) defined by the Institute of Electrical and Electronics Engineers (IEEE). Any device may be accepted as the user equipment 200 as long as the device can transmit and receive data to and from the blood sugar management server 300 and perform operations based on the transmitted and received data.

For example, the user equipment 100 may be, but is not limited to, any one of fixed computing devices such as a desktop 200c, a workstation, and a server, and mobile computing devices such as a smartphone 200a, a laptop 200b, a tablet, a phablet, a portable multimedia player (PMP), a personal digital assistant (PDA), and an e-book reader.

As the next component, the blood sugar management server 300 may estimate the user's blood sugar level, glycated hemoglobin level, and/or the like based on the electrocardiogram 10 measured by the electrocardiogram reader 100, and may transmit the information about the guidance 20 on blood sugar or diabetes to the user equipment 200 based on the results of the estimation.

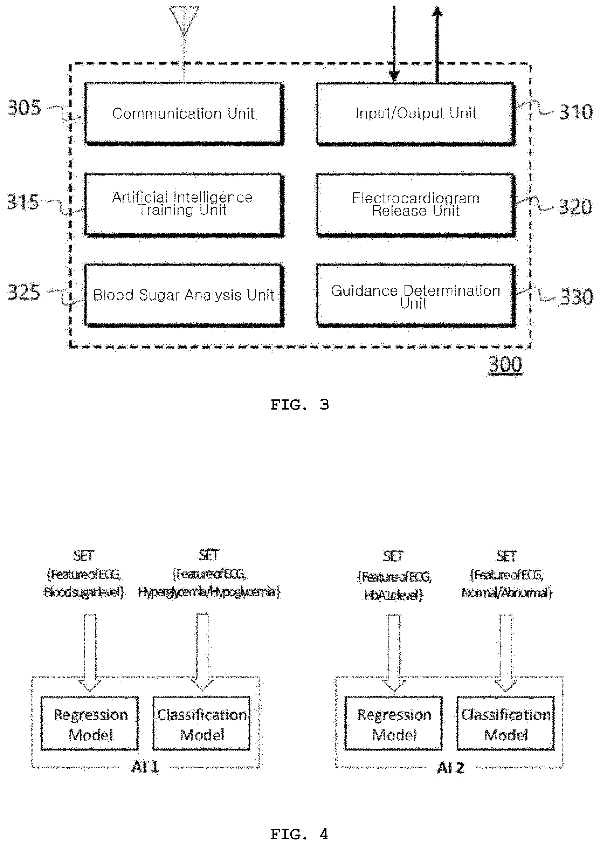

The specific components and operations of the blood sugar management server 300 will be described later with reference to FIGS. 3 to 12.

The one or more electrocardiogram readers 100, the one or more pieces of user equipment 200, and the blood sugar management server 300 that constitute the blood sugar management system as described above may transmit and receive data using a network, obtained by combining one or more of a secure line, a public wired communication network, and a mobile communication network, that directly connects the devices.

For example, the public wired communication network may include, but is not limited to, Ethernet, Digital Subscriber Line (xDSL), Hybrid Fiber Coax (HFC), and Fiber To The Home (FTTH) networks. Furthermore, the mobile communication network may include, but is not limited to, Code Division Multiple Access (CDMA), Wideband CDMA (WCDMA), High Speed Packet Access (HSPA), Long Term Evolution (LTE), and 5th generation mobile telecommunication networks.

The components of the blood sugar management server 300 having the features described above will be specifically described below.

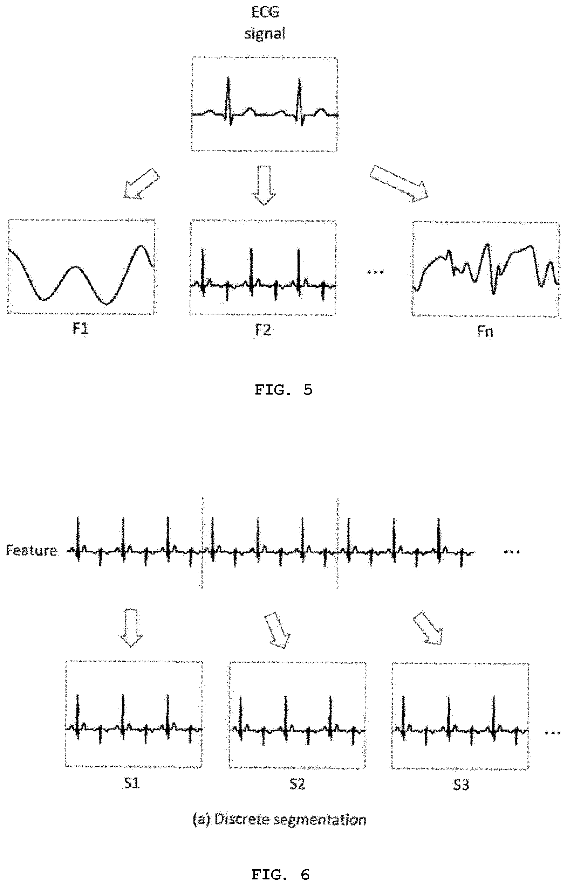

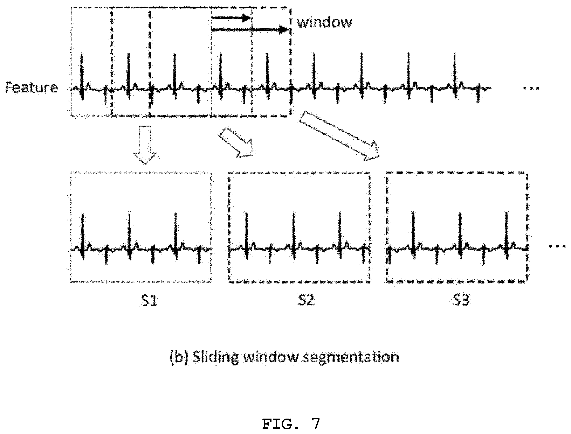

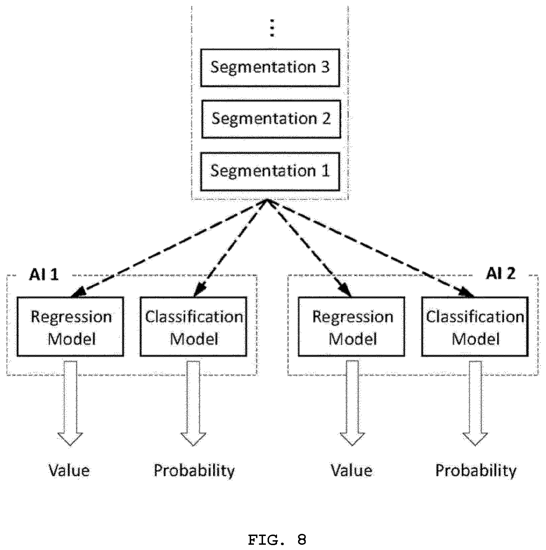

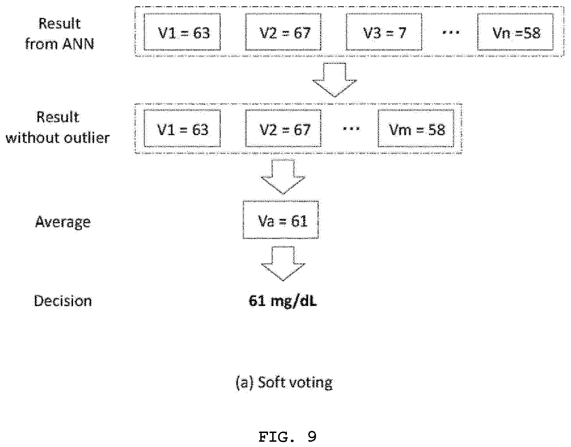

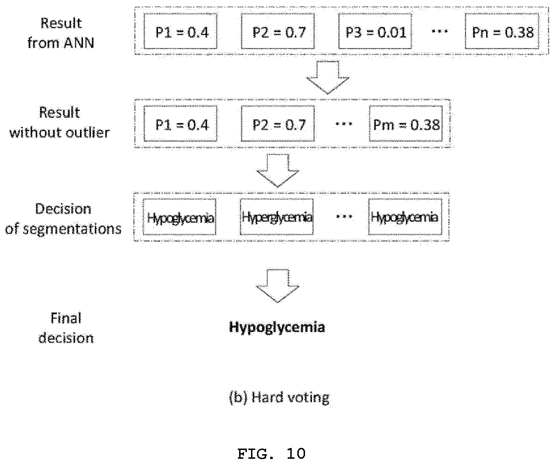

FIG. 3 is a diagram of the logical configuration of a blood sugar management server according to one embodiment of the present invention. FIG. 4 is an exemplary diagram illustrating a process of training an artificial intelligence (AI) according to one embodiment of the present invention. FIG. 5 is an exemplary diagram illustrating a method of releasing an electrocardiogram signal according to one embodiment of the present invention. FIGS. 6 and 7 are exemplary diagrams illustrating processes of performing segmentation into a plurality of segmentation units according to some embodiments of the present invention. FIG. 8 is an exemplary diagram illustrating processes of performing analysis using an artificial intelligence according to some embodiments of the present invention. Furthermore, FIGS. 9 and 10 are exemplary diagrams illustrating a process of interpreting the analysis results of artificial intelligences (AIs) according to one embodiment of the present invention.

As shown in FIG. 3, the blood sugar management server 300 according to one embodiment of the present invention may be configured to include a communication unit 305, an input/output unit 310, an artificial intelligence training unit 315, an electrocardiogram release unit 320, a blood sugar analysis unit 325, and a guidance determination unit 330.

The components of the blood sugar management server 300 according to the one embodiment of the present invention are merely functionally distinct components, so that two or more components may be implemented as an integrated component in an actual physical environment or one component may be implemented as separate components in an actual physical environment.

As for the individual components, the communication unit 305 may transmit and receive data to and from the electrocardiogram reader 100 or the user equipment 200.

More specifically, the communication unit 305 may receive an electrocardiogram (10) signal from the electrocardiogram reader 100. Furthermore, the communication unit 305 may transmit the information about the guidance 20 on blood sugar or diabetes to the user equipment 200.

As the next component, the input/output unit 310 may receive commands from an administrator or output operation results via a user interface (UI).

More specifically, the input/output unit 310 may receive a conversion method for the extraction of feature information from the electrocardiogram (10) signal. The input/output unit 310 may receive the size of a window, which is a criterion for the segmentation of feature information into a plurality of segmentation units. The input/output unit 310 may receive a validated range, which is a criterion for the removal of outliers from the probability values obtained from artificial intelligences (AIs).

The input/output unit 310 may receive a blood sugar level, which is a criterion for the determination of whether the user has hyperglycemia or hypoglycemia. Furthermore, the input/output unit 310 may receive a glycated hemoglobin (HbA1c) level, which is a criterion for the determination of whether the glycated hemoglobin (HbA1c) is normal or abnormal.

In addition, the input/output unit 310 may output the user's electrocardiogram (10) signal. The input/output unit 310 may also output information about guidance 20 on blood sugar or diabetes to be provided to the user.

As the next component, the artificial intelligence training unit 315 may cause a first artificial intelligence (AI) and a second artificial intelligence (AI) to be subjected to machine learning based on electrocardiogram (10) signals.

More specifically, the first artificial intelligence (AI) is an artificial intelligence (AI) for estimating the user's blood sugar level and determining whether the user has hyperglycemia or hypoglycemia. Furthermore, the second artificial intelligence (AI) is an artificial intelligence (AI) for estimating the user's glycated hemoglobin (HbA1c) level and determining whether the user's glycated hemoglobin (HbA1c) is normal or abnormal.

Referring to FIG. 4, the first artificial intelligence (AI) may include a multiple linear regression model for estimating the user's blood sugar level and a classification model for determining whether the user has hyperglycemia or hypoglycemia. Furthermore, the second artificial intelligence (AI) may include a multiple linear regression model for estimating the user's glycated hemoglobin (HbA1c) level and a classification model for determining whether the glycated hemoglobin (HbA1c) is normal or abnormal.

The artificial intelligence training unit 315 may set desired weights and biases for the multiple linear regression model of the first artificial intelligence (AI) based on a dataset of blood sugar levels and the feature vectors of the initial segmentation signals obtained through the segmentation of the electrocardiogram (10) signals input for supervised learning. The artificial intelligence training unit 315 may obtain result values by inputting the feature vectors of the segmentation signals, obtained through subsequent segmentation for supervised learning, to the multiple linear regression model of the first artificial intelligence (AI) with the desired weights and biases set therefor. The artificial intelligence training unit 315 may calculate a loss function between the feature vectors of the segmentation signals obtained through the subsequent segmentation, the blood sugar levels constituting the dataset and the result values obtained from the multiple linear regression model. Furthermore, the artificial intelligence training unit 315 may train the multiple linear regression model of the first artificial intelligence (AI) by updating the weights and the biases so that the error attributable to the loss function is minimized.

The artificial intelligence training unit 315 may set desired weights and biases for the classification model of the first artificial intelligence (AI) based on a dataset of the feature vectors of the initial segmentation signals, obtained through the segmentation of the electrocardiogram (10) signals input for supervised learning, and hyperglycemia or hypoglycemia classification results. The artificial intelligence training unit 315 may obtain result values by inputting the feature vectors of the segmentation signals, obtained through subsequent segmentation for supervised learning, to the classification model of the first artificial intelligence (AI) with the desired weights and biases set therefor. The artificial intelligence training unit 315 may calculate a loss function between the feature vectors of the segmentation signals obtained through the subsequent segmentation, the hyperglycemia or hypoglycemia classification results constituting the dataset, and the result values obtained from the classification model. Furthermore, the artificial intelligence training unit 315 may train the classification model of the first artificial intelligence (AI) by updating the weights and the biases so that the error attributable to the loss function is minimized.

The artificial intelligence training unit 315 may set desired weights and biases for the multiple linear regression model of the second artificial intelligence (AI) based on a dataset of the feature vectors of the initial segmentation signals, obtained through the segmentation of the electrocardiogram (10) signals input for supervised learning, and glycated hemoglobin (HbA1c) levels. The artificial intelligence training unit 315 may obtain result values by inputting the feature vectors of the segmentation signals, obtained through subsequent segmentation for supervised learning, to the multiple linear regression model of the second artificial intelligence (AI) with the desired weights and biases set therefor. The artificial intelligence training unit 315 may calculate a loss function between the feature vectors of the segmentation signals obtained through the subsequent segmentation, the glycated hemoglobin (HbA1c) levels constituting the dataset, and the result values obtained from the multiple linear regression model. Furthermore, the artificial intelligence training unit 315 may train the multiple linear regression model of the second artificial intelligence (AI) by updating the weights and the biases so that the error attributable to the loss function is minimized.

In addition, the artificial intelligence training unit 315 may set desired weights and biases for the classification model of the second artificial intelligence AI based on a dataset of the feature vectors of the initial segmentation signals, obtained through the segmentation of the electrocardiogram (10) signals input for supervised learning, and normal or abnormal classification results. The artificial intelligence training unit 315 may obtain result values by inputting the feature vectors of the segmentation signals, obtained through subsequent segmentation for supervised learning, to the classification model of the second artificial intelligence (AI) with the desired weights and biases set therefor. The artificial intelligence training unit 315 may calculate a loss function between the feature vectors of the segmentation signals obtained through the subsequent segmentation, the normal or abnormal classification results constituting the dataset, and the result values obtained from the classification model. Furthermore, the artificial intelligence training unit 315 may train the classification model of the second artificial intelligence (AI) by updating the weights and the biases so that the error attributable to the loss function is minimized.

In this case, the loss function used for training the first artificial intelligence (AI) or the second artificial intelligence (AI) may be the Mean Square Error (MSE), but is not limited thereto. It may be replaced with the Root Mean Squared Error (RMSE), the Cross Entropy Error (CEE), the Binary Cross Entropy Error (BCEE), the Categorical Cross Entropy Error (CCEE), or the Focal loss.

Furthermore, the artificial intelligence training unit 315 may update the weights and the biases according to the gradient descent method, but is not limited thereto.

Referring back to FIG. 3 to describe the next component, the electrocardiogram release unit 320 may release the electrocardiogram (10) signal measured by the electrocardiogram reader 100.

More specifically, the electrocardiogram release unit 320 may obtain the electrocardiogram (10) signal measured for the user. That is, the electrocardiogram release unit 320 may directly receive the electrocardiogram (10) signal from the electrocardiogram reader 100 via the communication unit 305, or may directly receive the electrocardiogram (10) signal via the input/output unit 310.

As shown in FIG. 5, the electrocardiogram release unit 320 may extract at least one piece of feature information F1, F2, ..., Fn by decomposing the obtained electrocardiogram (10) signal based on at least one of amplitude, frequency, and time.

For example, the electrocardiogram release unit 320 may extract feature information F1, F2, ..., Fn by decomposing the electrocardiogram (10) signal using one of wavelet transform, Ensemble Empirical Mode Decomposition (EEMD), Triadic Motif Field (TMF), and Triadic Motif Difference Field (TMDF). The method by which the electrocardiogram release unit 320 decomposes the electrocardiogram (10) signal is not limited thereto.

Thereafter, the electrocardiogram release unit 320 may segment the extracted feature information F1, F2, ..., Fn into a plurality of segmentation units according to the time-series order in which the electrocardiogram signal was measured from the user.

Basically, as shown in FIG. 6, the electrocardiogram release unit 320 may segment each piece of feature information F1, F2, ..., Fn into a plurality of separate segmentation units S1, S2, S3, ... by discretely cutting it at a predetermined size without overlap areas. In this case, the size at which each piece of feature information F1, F2, ..., Fn is cut discretely may be a predetermined value set by the input/output unit 310, but is not limited thereto. It may also be a randomized value.

In contrast, as shown in FIG. 7, the electrocardiogram release unit 320 may segment each piece of feature information F1, F2, ..., Fn into a plurality of segmentation units S1, S2, S3, ... each having a size corresponding to that of the window while sliding the window along the time axis. In this case, the size of the window for the cutting of each piece of feature information F1, F2, ..., Fn may be a specific value preset by the input/output unit 310.

Meanwhile, the electrocardiogram (10) signal may include the noise generated by the user's behavior during a measurement process. Accordingly, the electrocardiogram release unit 320 may remove the noise included in the plurality of segmentation signals S1, S2, S3, ....

More specifically, the electrocardiogram release unit 320 may identify displacements for keypoints included in each of the plurality of segmentation signals S1, S2, S3, **....** The electrocardiogram release unit 320 may generate a normal distribution of the displacements for the keypoints. Furthermore, the electrocardiogram release unit 320 may selectively remove only segmentation signals, having the keypoints included in a preset noise range, from the generated normal distribution. In this case, a method of identifying keypoints from the plurality of segmentation units S1, S2, S3, ... may follow preset keypoint extraction criteria, but is not limited thereto.

Referring back to FIG. 3 to describe the next component, the blood sugar analysis unit 325 may estimate the user's blood sugar level by analyzing the electrocardiogram (10) signal using the first artificial intelligence (AI) pre-trained via the artificial intelligence training unit 315, and may determine whether the user has hyperglycemia or hypoglycemia by analyzing the electrocardiogram (10) signal using the artificial intelligence (AI).

In addition, the blood sugar analysis unit 325 may additionally estimate the user's glycated hemoglobin (HbA1c) level by analyzing the electrocardiogram (10) signal using the second artificial intelligence (AI) pre-trained via the artificial intelligence training unit 315, and may additionally determine whether the glycated hemoglobin (HbA1c) is normal or abnormal by analyzing the electrocardiogram (10) signal using the artificial intelligence (AI).

First, the blood sugar analysis unit 325 may obtain pluralities of level values V1, V2, V3, ..., Vn and probability values P1, P2, P3, ..., Pn from the first and second artificial intelligences (AIs) by inputting the plurality of segmentation units S1, S2, S3, ..., obtained through the segmentation of the electrocardiogram release unit 320, to each of the first and second artificial intelligences.

More specifically, the blood sugar analysis unit 325 may identify feature vectors for the plurality of segmentation units S1, S2, S3, ... obtained through the segmentation of the electrocardiogram release unit 320.

As shown in FIG. 8, the blood sugar analysis unit 325 may input the identified feature vectors to each of the multiple linear regression model and classification model of the first artificial intelligence (AI). Furthermore, the blood sugar analysis unit 325 may input the identified feature vectors to each of the multiple linear regression model and classification model of the second artificial intelligence (AI).

In addition, the blood sugar analysis unit 325 may obtain the plurality of level values V1, V2, V3, ..., Vn for the estimation of blood sugar levels from the multiple linear regression model of the first artificial intelligence (AI). The blood sugar analysis unit 325 may obtain the plurality of probability values P1, P2, P3, ..., Pn for the determination of whether the user has hyperglycemia or hypoglycemia from the classification model of the first artificial intelligence (AI). The blood sugar analysis unit 325 may obtain the plurality of level values V1, V2, V3, ..., Vn for the estimation of glycated hemoglobin (HbA1c) levels from the multiple linear regression model of the second artificial intelligence (AI). The blood sugar analysis unit 325 may obtain the plurality of probability values P1, P2, P3, ..., Pn for the determination of whether the glycated hemoglobin (HbA1c) is normal or abnormal from the classification model of the second artificial intelligence (AI).

Next, the blood sugar analysis unit 325 may estimate the user's blood sugar level and determine whether the user has hyperglycemia or hypoglycemia based on the level values and probability values obtained from the first artificial intelligence (AI). Furthermore, the blood sugar analysis unit 325 may estimate the user's glycated hemoglobin (HbA1c) level and determine whether the glycated hemoglobin (HbA1c) is normal or abnormal based on the level values and probability values obtained from the second artificial intelligence (AI).

As shown in FIG. 9, the blood sugar analysis unit 325 may remove outliers, included in the values V1, V2, V3, ..., Vn obtained from the first and second artificial intelligences (AIs), based on a preset validated range.

The blood sugar analysis unit 325 may estimate the user's blood sugar level by using the result of soft voting based on the average value of the level values V1, V2, ..., Vm from which outliers have been removed among the level values V1, V2, V3, ..., Vn obtained from the first artificial intelligence (AI).

The blood sugar analysis unit 325 may estimate the user's glycated hemoglobin (HbA1c) level by using the result of soft voting based on the average value of the level values V1, V2, ..., Vm from which outliers have been removed among the level values V1, V2, V3, ..., Vn obtained from the second artificial intelligence (AI).

As shown in FIG. 10, the blood sugar analysis unit 325 may remove the outliers, included in the probability values P1, P2, P3, ..., Pn obtained from the first and second artificial intelligences AI, based on the preset validated range.

The blood sugar analysis unit 325 may determine whether the user has hypoglycemia or hyperglycemia by using the result of hard voting based on the majority of the level values P1, P2, ..., Pm from which outliers have been removed among the probability values P1, P2, P3, ..., Pn obtained from the first artificial intelligence (AI).

The blood sugar analysis unit 325 may determine whether the glycated hemoglobin (HbA1c) is normal or abnormal by using the result of hard voting based on the majority of the probability values P1, P2, P3, ..., Pm from which outliers are removed among the probability values P1, P2, P3, ..., Pn obtained from the second artificial intelligence (AI).

Referring back to FIG. 3 to describe the next component, the guidance determination unit 330 may determine whether the user needs guidance on blood sugar or diabetes based on the blood sugar level estimated by the blood sugar analysis unit 325 and the results of the determination of whether the user has hyperglycemia or hypoglycemia. Furthermore, the guidance determination unit 330 may determine whether the user needs guidance on blood sugar or diabetes by additionally taking into consideration the glycated hemoglobin (HbA1c) level estimated by the blood sugar analysis unit 325 and the results of the determination of whether the glycated hemoglobin (HbA1c) is normal or abnormal.

More specifically, the guidance determination unit 330 may primarily determine whether the user has hyperglycemia or hypoglycemia based on the blood sugar level estimated using the multiple linear regression model of the first artificial intelligence (AI). The guidance determination unit 330 may secondarily determine whether the results of the primary determination are the same as the results of classification obtained via the classification model of the first artificial intelligence (AI). Furthermore, the guidance determination unit 330 may determine whether the user needs guidance on blood sugar based on the results of the secondary determination.

For example, when the blood sugar level estimated using the multiple linear regression model of the first artificial intelligence (AI) is 65 mg/dL or lower and is classified as hypoglycemia via the classification model of the first artificial intelligence (AI), the guidance determination unit 330 may determine that the user needs guidance on blood sugar. In contrast, when the blood sugar level estimated using the multiple linear regression model of the first artificial intelligence (AI) is 95 mg/dL or higher and classified as hyperglycemia via the classification model of the first artificial intelligence (AI), the guidance determination unit 330 may determine that the user needs guidance on blood sugar.

The guidance determination unit 330 may primarily determine whether the glycated hemoglobin (HbA1c) is normal or abnormal based on the glycated hemoglobin (HbA1c) level estimated using the multiple linear regression model of the second artificial intelligence (AI). The guidance determination unit 330 may secondarily determine whether the results of the primary determination are the same as the results obtained via the classification of the classification model of the second artificial intelligence (AI). Furthermore, the guidance determination unit 330 may determine whether the user needs guidance on the management of diabetes based on the results of the secondary determination.

For example, when the glycated hemoglobin (HbA1c) level estimated using the multiple linear regression model of the second artificial intelligence (AI) is 5.7% or higher and is classified as abnormal via the classification model of the second artificial intelligence (AI), the guidance determination unit 330 may determine that the user needs guidance on the management of diabetes.

Meanwhile, when the results of the primary determination based on the level values of the multiple linear regression model are different from the results of classification via the probability values of the classification model, the artificial intelligence training unit 315 may readjust the weights and biases set for the multiple linear regression model or the classification model of the first and second artificial intelligences (AI) so that the matching rate between the primary determination based on the level values of the multiple linear regression model and the results of classification via the probability values of the classification model falls within a preset validated range.

Thereafter, when it is determined that the user needs guidance on blood sugar or diabetes, the guidance determination unit 330 may set information about guidance 20 on blood sugar or diabetes based on the user's blood sugar level, the user's glycated hemoglobin (HbA1c) level, the result of the determination of whether the user has hyperglycemia or hypoglycemia, and/or the result of the determination of whether the glycated hemoglobin (HbA1c) is normal or abnormal.

In this case, the information about guidance 20 on blood sugar or diabetes may directly include the blood sugar level, the glycated hemoglobin (HbA1c) level, the result of the determination of whether the user has hyperglycemia or hypoglycemia, or the result of the determination of whether glycated hemoglobin (HbA1c) is normal or abnormal, or may include indirect precautions, recommendations, or warnings based on the blood sugar level, the glycated hemoglobin (HbA1c) level, the result of whether hyperglycemia or hypoglycemia is present, or the result of whether glycated hemoglobin (HbA1c) is normal or abnormal.

Furthermore, the information on guidance 20 on blood sugar or diabetes may include at least one of guidance on actions that the user has to take immediately in connection with blood sugar or diabetes and guidance intended to support the user's management of diabetes.

For example, the guidance determination unit 330 may set guidance on the initial measures that the user has to take immediately based on the user's blood sugar level and whether the user has hyperglycemia or hypoglycemia.

Furthermore, the guidance determination unit 330 may set guidance intended to support the user's management of diabetes based on the user's glycated hemoglobin (HbA1c) level and whether the user's glycated hemoglobin (HbA1c) is normal or abnormal.

The guidance determination unit 330 may identify a human weight preset to correspond to the user's gender and age. The guidance determination unit 330 may determine a distance weight to correspond to a distance value from the location of the user equipment 200 to a medical institution located at the shortest distance. The guidance determination unit 330 may determine the tone of sentences constituting the guidance 20 on blood sugar or diabetes based on the identified human weight and the determined distance weight. Furthermore, the guidance determination unit 330 may construct information about the guidance 20 on blood sugar or diabetes according to the determined tone of sentences.

In addition, the guidance determination unit 330 may transmit the set information about the guidance 20 on blood sugar or diabetes to the user equipment 200 preset to correspond to the user.

Hardware for implementing the logical components of the blood sugar management server 300 having the features described above will be described in more detail below.

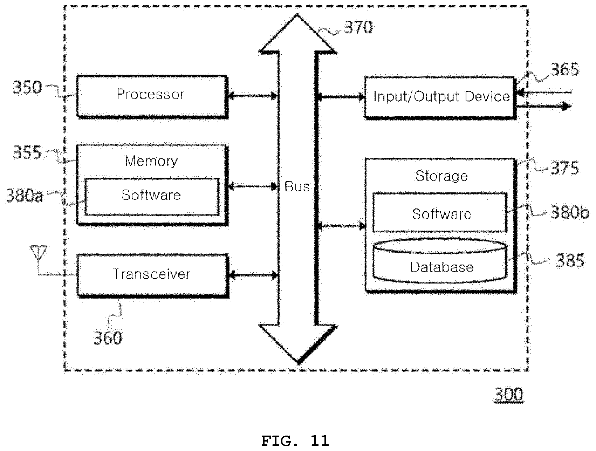

FIG. 11 is a diagram of the hardware configuration of a blood sugar management server according to one embodiment of the present invention.

As shown in FIG. 9, the blood sugar management server 300 according to the one embodiment of the present invention may be configured to include a processor 350, memory 355, a transceiver 360, an input/output device 365, a data bus 370, and storage 375.

More specifically, the processor 350 may implement the operations and functions of the blood sugar management server 300 based on instructions according to software 380a implementing a method of measuring the real-time level and degree of management of blood sugar that has been loaded into the memory 355.

The memory 355 may be loaded with software 380b implementing the method of measuring the real-time level and degree of management of blood sugar that is stored in the storage 375.

The transceiver 360 may transmit and receive data to and from one or more of the electrocardiogram reader 100 and the user equipment 200.

The input/output device 365 may receive the signals required for the operation of the blood sugar management server 300 or output operation results to the outside according to the commands of the processor 350.

The data bus 370 may be connected to the processor 350, the memory 355, the transceiver 360, the input/output device 365, and the storage 375, and may serve as a passage for the transmission of signals between the individual components.

The storage 375 may store application programming interfaces (APIs), library files, and resource files required for executing the software 380a implementing methods of measuring the real-time level and degree of management of blood sugar according to various embodiments of the present invention. The storage 375 may store the software 380b implementing the methods of measuring the real-time level and degree of management of blood sugar according to the various embodiments of the present invention. Furthermore, the storage 375 may include a database 385 for storing artificial intelligence training data, and various setting values.

According to one embodiment of the present invention, software 380a and 380b for implementing a method of measuring the real-time level and degree of management of blood sugar, which is loaded into the memory 355 or stored in storage 375, may be a computer program recorded on a storage medium in order to execute the step of obtaining, by the processor 350, an electrocardiogram (10) signal measured for a user via the transceiver 360 or input/output device 365, the step of estimating, by the processor 350, the user's blood sugar level by analyzing the electrocardiogram signal using the pre-trained first artificial intelligence (AI), and determining, by the processor 350, whether the user has hyperglycemia or hypoglycemia by analyzing the electrocardiogram (10) signal using the artificial intelligence (AI), and the step of determining, by the processor 350, whether the user needs guidance 20 on blood sugar or diabetes based on the estimated blood sugar level and the result of the determination of whether the user has hyperglycemia or hypoglycemia.

More specifically, the processor 350 may be configured to include one or more of a Central Processing Unit (CPU), an Application-Specific Integrated Circuit (ASIC), a chipset, and a logic circuit, but is not limited thereto.

The memory 355 may be configured to include one or more of Read-Only Memory (ROM), Random Access Memory (RAM), flash memory, and a memory card, but is not limited thereto.

The input/output device 365 may be configured to include one or more of input devices such as buttons, switches, a keyboard, a mouse, a joystick, and a touch screen, and one or more of output devices such as a liquid crystal display (LCD), a light emitting diode (LED) display, an organic light emitting diode (OLED) display, an active matrix OLED (AMOLED) display, a printer, and a plotter, but is not limited thereto.

When the embodiments included herein are implemented as software, the above-described method may be implemented as modules (processes, functions, and/or the like) that perform the above-described functions. The individual modules may be loaded into the memory 355 and executed by the processor 350. The memory 355 may be present inside or outside the processor 350, and may be connected to the processor 350 via various means known to the public.

The individual components shown in FIG. 11 may be implemented by various means (e.g., hardware, firmware, software, or a combination thereof). When implemented by hardware, one embodiment of the present invention may be implemented by one or more Application Specific Integrated Circuits (ASICs), Digital Signal Processors (DSPs), Digital Signal Processing Devices (DSPDs), Programmable Logic Devices (PLDs), Field Programmable Gate Arrays (FPGAs), processors, controllers, microcontrollers, microprocessors, and/or the like.

Furthermore, when implemented by firmware or software, one embodiment of the present invention may be implemented in the form of modules, procedures, functions, and/or the like that perform the functions or operations described above, and may be recorded on a storage medium that can be read via various computer means. In this case, the storage medium may include program instructions, data files, data structures, and the like alone or in combination.

The program instructions recorded on the storage medium may be those specifically designed and configured for the present invention, or may be those known and available to those having ordinary knowledge in the computer software industry. For example, the storage medium includes magnetic media such as a hard disk, a floppy disk, and magnetic tape, optical media such as compact disk read-only memory (CD-ROM) and a digital video disk (DVD), magneto-optical media such as a floptical disk, and hardware devices specifically configured to store and execute program instructions such as ROM, RAM, and flash memory.

Examples of the program instructions may include not only machine language codes such as those that are generated by a compiler, but also high-level language codes that can be executed by a computer using an interpreter or the like. Such hardware devices may be configured to operate as one or more pieces of software to perform the operations of the present invention, and vice versa.

The operation of the blood sugar management server 300 described above will be specifically described below.

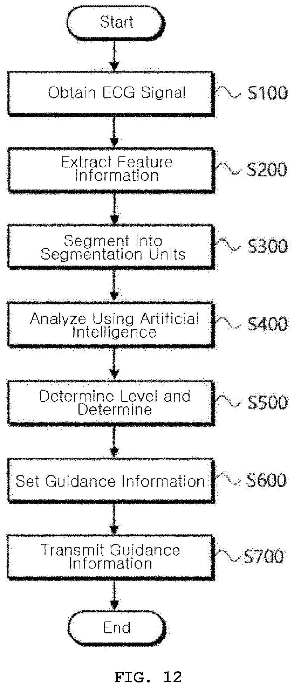

FIG. 12 is a flowchart illustrating a method of measuring the real-time level and degree of management of blood sugar according to one embodiment of the present invention.

As shown in FIG. 12, the blood sugar management server 300 according to one embodiment of the present invention may obtain an electrocardiogram (10) signal measured for a user in step S100.

More specifically, the blood sugar management server 300 may directly receive the electrocardiogram (10) signal from the electrocardiogram reader 100, or may directly receive the signal from the user or someone else.

Thereafter, the blood sugar management server 300 may extract at least one piece of feature information F1, F2, ..., Fn by decomposing the obtained electrocardiogram (10) signal based on at least one of amplitude, frequency, and time in step S200.

For example, the blood sugar management server 300 may extract feature information F1, F2, ..., Fn by decomposing the electrocardiogram (10) signal using one of wavelet transform, Ensemble Empirical Mode Decomposition (EEMD), Triadic Motif Field (TMF), and Triadic Motif Difference Field (TMDF), but the method by which the blood sugar management server 300 decomposes the electrocardiogram (10) signal is not limited thereto.

Thereafter, the blood sugar management server 300 may segment the extracted feature information F1, F2, ..., Fn into a plurality of segmentation units according to the time-series order in which the electrocardiogram signal was measured from the user in step S300.

According to one embodiment, the blood sugar management server 300 may segment each piece of feature information F1, F2, ..., Fn into a plurality of separate segmentation units S1, S2, S3, ... by discretely cutting it at a predetermined size without overlap areas. According to another embodiment, the blood sugar management server 300 may segment each piece of feature information F1, F2, ..., Fn into a plurality of segmentation units S1, S2, S3, ... each having a size corresponding to that of a window while sliding the window along the time axis.

Thereafter, the blood sugar management server 300 may estimate the user's blood sugar level and determine whether the user has hyperglycemia or hypoglycemia by analyzing the electrocardiogram (10) signal using the pre-trained first artificial intelligence (AI). Furthermore, the blood sugar management server 300 may additionally estimate the user's glycated hemoglobin (HbA1c) level and additionally determine whether the glycated hemoglobin (HbA1c) is normal or abnormal by analyzing the electrocardiogram (10) signal using the pre-trained second artificial intelligence (AI) in step S400.

More specifically, the blood sugar management server 300 may obtain pluralities of level values V1, V2, V3, ..., Vn and probability values P1, P2, P3, ..., Pn from the first and second artificial intelligences (AIs) by inputting the plurality of segmentation units S1, S2, S3, ... to each of the first and second artificial intelligences.

Thereafter, the blood sugar management server 300 may estimate the user's blood sugar level and determine whether the user has hyperglycemia or hypoglycemia based on the level values and probability values obtained from the first artificial intelligence (AI). Furthermore, the blood sugar management server 300 may estimate the user's glycated hemoglobin (HbA1c) level and determine whether the glycated hemoglobin (HbA1c) is normal or abnormal based on the level values and probability values obtained from the second artificial intelligence (AI) in step S500.

More specifically, the blood sugar management server 300 may remove outliers, included in the level values V1, V2, V3, ..., Vn obtained from the first and second artificial intelligences (AIs), based on a preset validated range.

The blood sugar management server 300 may estimate the user's blood sugar level using the result of soft voting based on the average value of the level values V1, V2, ..., Vm from which outliers have been removed among the level values V1, V2, V3, ..., Vn obtained from the first artificial intelligence (AI), and may estimate the user's glycated hemoglobin (HbA1c) level by using the result of soft voting based on the average value of the level values V1, V2, ..., Vm from which outliers have been removed among the level values V1, V2, V3, ..., Vn obtained from the second artificial intelligence (AI).

The blood sugar management server 300 may remove the outliers, included in the probability values P1, P2, P3, ..., Pn obtained from the first and second artificial intelligences AI, based on the preset validated range.

In addition, the blood sugar management server 300 may determine whether the user has hypoglycemia or hyperglycemia by using the result of hard voting based on the majority of the level values P1, P2, ..., Pm from which outliers have been removed among the probability values P1, P2, P3, ..., Pn obtained from the first artificial intelligence (AI), and may determine whether the glycated hemoglobin (HbA1c) is normal or abnormal by using the result of hard voting based on the majority of the probability values P1, P2, P3, ..., Pm from which outliers are removed among the probability values P1, P2, P3, ..., Pn obtained from the second artificial intelligence (AI).

Thereafter, the blood sugar management server 300 may set information about guidance 20 on blood sugar or diabetes based on the user's blood sugar level, the user's glycated hemoglobin (HbA1c) level, the result of the determination of whether the user has hyperglycemia or hypoglycemia, and/or the determination of whether the glycated hemoglobin (HbA1c) is normal or abnormal in step S600.

More specifically, the blood sugar management server 300 may determine whether the user needs guidance on blood sugar based on the blood sugar level and the result of determination of whether blood sugar is high or low, and may also determine whether the user needs guidance on diabetes based on the glycated hemoglobin (HbA) level and the result of determination of whether blood sugar is high or low.

In addition, the information about the guidance 20 on blood sugar or diabetes may directly include the blood sugar level, the glycated hemoglobin (HbA1c) level, the result of whether the blood sugar is high or low, and/or the result of whether the glycated hemoglobin (HbA1c) is normal or abnormal, or may include indirect precautions, recommendations, and/or warnings based on the blood sugar level, the glycated hemoglobin (HbA1c) level, the result of whether the blood sugar is high or low, and/or the result of whether the glycated hemoglobin (HbA1c) is normal or abnormal. Furthermore, the information about the guidance 20 on blood sugar or diabetes may include at least one of guidance on actions that the user has to take immediately in connection with blood sugar or diabetes and guidance intended to support the user's diabetes management.

Finally, the blood sugar management server 300 may transmit the set information about the guidance 20 on blood sugar or diabetes to the user equipment 200 preset to correspond to the user in step S700.

As described above, although the preferred embodiments of the present invention have been disclosed in the present specification and the accompanying drawings, it is obvious to those skilled in the art that other modified examples based on the technical spirit of the present invention can be implemented in addition to the embodiments disclosed herein. Furthermore, although specific terms have been used in the present specification and the accompanying drawings, they have been used only in general senses to easily describe the technical content of the present invention and to help the understanding of the present invention, and are not intended to limit the scope of the present invention. Therefore, the detailed description above should not be construed as restrictive and should be considered as exemplary in all aspects. The scope of the present invention should be determined by a reasonable interpretation of the appended claims, and all changes within the equivalent scope of the present invention are included in the scope of the present invention.

## Claims

1. A method of measuring a real-time level and degree of management of blood sugar, the method comprising:
obtaining an electrocardiogram signal measured for a user;
estimating the user's blood sugar level by analyzing the electrocardiogram signal using a pre-trained first artificial intelligence, and determining whether the user has hyperglycemia or hypoglycemia by analyzing the electrocardiogram signal using an artificial intelligence; and
determining whether the user needs guidance on blood sugar or diabetes based on the estimated blood sugar level and a result of the determination of whether the user has hyperglycemia or hypoglycemia.

2. The method of claim 1, wherein determining whether the user has hyperglycemia or hypoglycemia comprises estimating the user's glycated hemoglobin (HbA1c) level by analyzing the electrocardiogram signal using a pre-trained second artificial intelligence and determining whether glycated hemoglobin (HbA1c) is normal or abnormal by analyzing the electrocardiogram signal using the second artificial intelligence.

3. The method of claim 2, wherein determining whether the user has hyperglycemia or hypoglycemia comprises:
extracting at least one piece of feature information by decomposing the obtained electrocardiogram signal based on at least one of amplitude, frequency, and time;
segmenting the extracted feature information into a plurality of segmentation units according to a time-series order in which the electrocardiogram signal was measured;
obtaining pluralities of level values and probability values from the first and second artificial intelligences by inputting the plurality of segmentation units into each of the first and second artificial intelligences; and
estimating the blood sugar level and determining whether the user has hyperglycemia or hypoglycemia based on the level values and probability values obtained from the first artificial intelligence, and estimating the glycated hemoglobin (HbA1c) level and determining whether glycated hemoglobin (HbA1c) is normal or abnormal based on the level values and probability values obtained from the second artificial intelligence.

4. The method of claim 3, wherein extracting the feature information comprises extracting the feature information by decomposing the electrocardiogram signal using one of wavelet transform, Ensemble Empirical Mode Decomposition (EEMD), Triadic Motif Field (TMF), and Triadic Motif Difference Field (TMDF) .

5. The method of claim 3, wherein segmenting the extracted feature information into the plurality of segmentation units comprises segmenting the feature information, obtained from the electrocardiogram signal, into a plurality of segmentation units each having a size corresponding to that of a window while sliding the window having a preset size along a time axis.

6. The method of claim 3, wherein obtaining the pluralities of level values and probability values comprises obtaining the pluralities of level values and probability values by inputting feature vectors for the plurality of segmentation units to each of multiple linear regression and classification models of the first and second artificial intelligences.

7. The method of claim 3, wherein determining whether the glycated hemoglobin (HbA1c) is normal or abnormal comprises removing outliers, included in the level values obtained from the first and second artificial intelligences, based on a preset validated range, estimating the blood sugar level by using a result of soft voting based on an average value of level values from which outliers have been removed among the level values obtained from the first artificial intelligence, and estimating the glycated hemoglobin (HbA1c) level by using a result of soft voting based on an average value of level values from which outliers have been removed among the level values obtained from the second artificial intelligence.

8. The method of claim 3, wherein determining whether the glycated hemoglobin (HbAlc) is normal or abnormal comprises removing outliers, included in the probability values obtained from the first and second artificial intelligences, based on a preset validated range, determining whether the user has hypoglycemia or hyperglycemia by using a result of hard voting based on a majority of level values from which outliers have been removed among the probability values obtained from the first artificial intelligence, and determining whether the glycated hemoglobin (HbAlc) is normal or abnormal by using a result of hard voting based on a majority of probability values from which outliers are removed among the probability values obtained from the second artificial intelligence.

9. The method of claim 3, further comprising, after determining whether the user needs guidance, when it is determined that the user needs guidance, transmitting information about guidance, set based on the blood sugar level, the glycated hemoglobin (HbAlc) level, whether the user has hyperglycemia or hypoglycemia, and/or whether the glycated hemoglobin (HbA1c) is normal or abnormal, to user equipment (UE) preset to correspond to the user.

10. A computer program recorded on a storage medium, which is coupled to a computing device, including:
memory;
a transceiver;
an input/output device; and
a processor for processing instructions loaded into the memory, in order to execute:
obtaining, by the processor, an electrocardiogram signal measured for a user via the transceiver or input/output device;
estimating, by the processor, the user's blood sugar level by analyzing the electrocardiogram signal using a pre-trained first artificial intelligence, and determining, by the processor, whether the user has hyperglycemia or hypoglycemia by analyzing the electrocardiogram signal using an artificial intelligence; and
determining, by the processor, whether the user needs guidance on blood sugar or diabetes based on the estimated blood sugar level and a result of the determination of whether the user has hyperglycemia or hypoglycemia.
